# EUROPEAN PATENT APPLICATION

(11) **EP 2 040 077 A1**
(43) Date of publication of application: **25.03.2009**
(21) Application number: 08016382.7
(22) Date of filing: 17.09.2008
(51) Int. Cl.: G01N 33/553, G01N 21/55

(54) **Method for producing an immobilization substrate and immobilization substrate produced by the method**

(30) Priority: 21.09.2007 JP 2007246047
(71) Applicant: FUJIFILM Corporation, Minato-ku Tokyo (JP)
(72) Inventor: Kuruma, Koji, Ashigarakami-gun Kanagawa (JP)
(74) Representative: HOFFMANN EITLE

(57) **Abstract**

The present invention provides a method for preparing an immobilization substrate for a biosensor, the immobilization substrate having a measurement surface having a physiologically active substance immobilized thereon, and a reference surface not having a physiologically active substance immobilized thereon, the biosensor measuring an interaction, in terms of a change in refractive index, between the physiologically active substance and a test substance in a solution which is supplied to both the measurement surface and the reference surface, the method comprising: performing an activation treatment on a part of the substrate surface by using an organic solvent and an activating agent, thereby forming, on the substrate, a first surface which is activated such that it can immobilize the physiologically active substance, and forming a second surface which has not been subjected to the activation treatment; forming a flow channel which includes both the first surface and the second surface by attaching a flow channel member to the substrate; and supplying the physiologically active substance to the flow channel to bring the first surface into contact with the physiologically active substance, and thereby prepare the measurement surface and the reference surface within the same flow channel; and an immobilization substrate produced by the above method for producing an immobilization substrate.

## Description

### BACKGROUND OF THE INVENTION

### Field of the invention

The present invention relates to a method for producing an immobilization substrate and immobilization substrate produced by the method.

### Description of the Related Art

Recently, a large number of measurements using intermolecular interactions such as immune responses are being carried out in clinical tests, etc. Among them, several techniques are preferably used that are capable of detecting the change in the binding amount of a test substance with high sensitivity. Examples of such a technique may include a surface plasmon resonance (SPR) measurement technique, a quartz crystal microbalance (QCM) measurement technique, and a measurement technique of using functional surfaces ranging from gold colloid particles to ultra-fine particles.

The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). Further, the ultra-fine particle surface (nm level) of gold is functionalized, and physiologically active substances are immobilized thereon. Thus, a reaction to recognize specificity among physiologically active substances is carried out, thereby detecting a substance associated with a living organism from sedimentation of gold fine particles or sequences.
In all of the above-described techniques, the surface where a physiologically active substance is immobilized is important. Surface plasmon resonance (SPR), which is most commonly used in this technical field, will be described below as an example.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules.

Japanese Patent No. 2,815,120 discloses in detail a method for producing hydrogel, which is used as a detection surface having a functional group capable of immobilizing a physiologically active substance, for example. Specifically, a 16-mercaptohexadecanol layer binds to a gold film, so as to form a barrier layer. In the subsequent step, dextran is layered on the barrier layer and a carboxymethyl group is introduced to the dexstran.
The surface of the carboxymethyl-modified dextran produced based on this method can immobilize a physiologically active substance having an amino group (e.g., a protein or an amino acid).

That is to say, a portion of carboxyl groups in carboxymethyl degeneration dextran are treated with an aqueous solution that contains N-hydroxysuccinimide (NHS) and N-(3-dimethylaminopropyl)-N'-ethylcarbodiimide (EDC) hydrochloride, for example, so that such groups are degenerated so as to achieve a reactive ester function. An aqueous solution of a physiologically active substance containing an amino group (a protein or amino acid) is allowed to come into contact with such a detection surface, so as to allow the physiologically active substance containing an amino group to bind to a dextran matrix via a covalent bond.

Generally, in the case of immobilizing a physiologically active substance to the surface of the dextran formed as described above, it becomes further possible to detect a substance (analyte) capable of binding to the physiologically active substance (ligand). In this case, the ligand is immobilized to form a measurement surface and, after a buffer which functions as a base is allowed to flow, a solution of the analyte is allowed to flow to thereby form ligand-analyte bond. This ligand-analyte bond is detected as a signal of change in the refractive index. In order to exclude other factors causing change in the refractive index than the bond formation, there is known a method of simultaneously measuring a signal of change in the refractive index on the reference surface on which no ligand is immobilized, and subtracting the value to correct.

In order to separately form the measurement surface and the reference surface, it is generally conducted to zone a uniform surface by a flow channel, and activate only a portion corresponding to the measurement surface. In this method, however, there is involved the problem that the structure for zoning to separately form the portion corresponding to the measurement surface and the portion corresponding to the reference surface becomes complicated.
On the other hand, there is a method of partly activating the substrate surface, attaching a flow channel member, and supplying a ligand-containing solution to the flow channel to thereby prepare the measurement surface. In general, however, the measurement surface or the reference surface is provided in one flow channel constituted by a pair of supplying inlet and discharging outlet, and hence it is required to supply the ligand-containing solution with selecting the flow channel, thus the procedure being troublesome. Further, with a commonly employed water-soluble activating agent, there is involved the problem that the activated portion becomes inactive with the lapse of time, and the amount of immobilized ligand is decreased with the lapse of time.

### SUMMARY OF THE INVENTION

The present invention has been made in view of the above circumstances and provides a method for producing an immobilization substrate and an immobilization substrate produced by the method.
A first aspect of the invention provides a method for preparing an immobilization substrate for a biosensor, the immobilization substrate having a measurement surface having a physiologically active substance immobilized thereon, and a reference surface not having a physiologically active substance immobilized thereon, the biosensor measuring an interaction, in terms of a change in refractive index, between the physiologically active substance and a test substance in a solution which is supplied to both the measurement surface and the reference surface, the method comprising: performing an activation treatment on a part of the substrate surface by using an organic solvent and an activating agent, thereby forming, on the substrate, a first surface which is activated such that it can immobilize the physiologically active substance, and forming a second surface which has not been subjected to the activation treatment; forming a flow channel which includes both the first surface and the second surface by attaching a flow channel member to the substrate; and supplying the physiologically active substance to the flow channel to bring the first surface into contact with the physiologically active substance, and thereby prepare the measurement surface and the reference surface within the same flow channel.
A second aspect of the invention provides an immobilization substrate produced by the above method for producing an immobilization substrate.

### BRIEF DESCRIPTION OF THE DRAWINGS

Fig. 1 is an exploded perspective view schematically showing the structure of the sensor chip in accordance with an embodiment of the present invention.
Fig. 2 is a schematic cross-sectional view of the sensor chip in accordance with an embodiment of the invention.

### RETAILED DESCRIPTION OF THE INVENTION

The method of the present invention for producing an immobilization substrate for a biosensor, the immobilization substrate having a measurement surface having a physiologically active substance immobilized thereon, and a reference surface not having a physiologically active substance immobilized thereon, the biosensor measuring an interaction, in terms of a change in refractive index, between the physiologically active substance and a test substance in a solution which is supplied to both the measurement surface and the reference surface, comprises: performing an activation treatment on a part of the substrate surface by using an organic solvent and an activating agent, thereby forming, on the substrate, a first surface which is activated such that it can immobilize the physiologically active substance, and forming a second surface which has not been subjected to the activation treatment; forming a flow channel which includes both the first surface and the second surface by attaching a flow channel member to the substrate; and supplying the physiologically active substance to the flow channel to bring the first surface into contact with the physiologically active substance, and thereby prepare the measurement surface and the reference surface within the same flow channel.

According to the present invention, the activation treatment for obtaining the measurement surface at which a physiologically active substance is immobilized is carried out by using both an activating agent and an organic solvent with respect to only a non-continuous part of the surface, and then attaching thereto a flow channel member. Generally, in microanalysis in which the interaction between a test substance and a physiologically active substance is measured in terms of change in refractive index, a physiologically active substance with even a small amount of deactivation should be excluded. However, in the present invention, the use of an organic solvent in the activation treatment allows immobilization of a physiologically active substance, imparted by the activating agent, to be maintained over a long period of time. Also, since both the first surface, which is subjected to the activation treatment, and the second surface, which is not subjected to the activation treatment, exist within the same flow channel, the measurement surface and the reference surface may be prepared from the first surface and the second surface respectively, by a single step of supplying a physiologically active substance, without the necessity of selecting a particular flow channel. Thus, an immobilization substrate that can stably immobilize a physiologically active substance can be prepared conveniently and efficiently.
The invention will be described below.

Additionally, in the invention, the term "step" indicates not only an independent step but may also indicate a step which cannot be discriminated clearly from other steps, as long as the intended effects of the step may be obtained.
Further, any notation for expressing numerical ranges in the invention indicates a range defined by the minimum and maximum values and includes the minimum and maximum values.
The term "biologically active substance" as used in the invention indicates a substance which relates to a living organism and indicates any substance that may exhibit physiological functions in vivo.

The biosensor of the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured.

In the immobilization substrate of the present invention, a metal surface or metal film can be used as a substrate. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above substrate, an interstitial layer consisting of chrome or the like may be provided between the substrate and a metal layer.

The film thickness of a metal film is not limited. When the metal film is used for a surface plasmon resonance biosensor, the thickness is preferably from 0.1 nm to 500 nm, and particularly preferably from 1 nm to 200 nm. If the thickness is 500 nm or less, the surface plasmon phenomena of a medium can be sufficiently detected. Moreover, when an interstitial layer consisting of chrome or the like is provided, the thickness of the interstitial layer is preferably from 0.1 nm to 10 nm.

Formation of a metal film may be carried out by common methods, and examples of such a method may include sputtering method, evaporation method, ion plating method, electroplating method, and nonelectrolytic plating method.

A metal_film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate.
When a substrate used in the present invention is used for a surface plasmon resonance biosensor, examples of such a substrate may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used.

The above-mentioned substrate is fixed to a dielectric block of a measuring chip to be unified and constitute a sensor chip. The sensor unit may be exchangeably formed. An example thereof will be shown below.

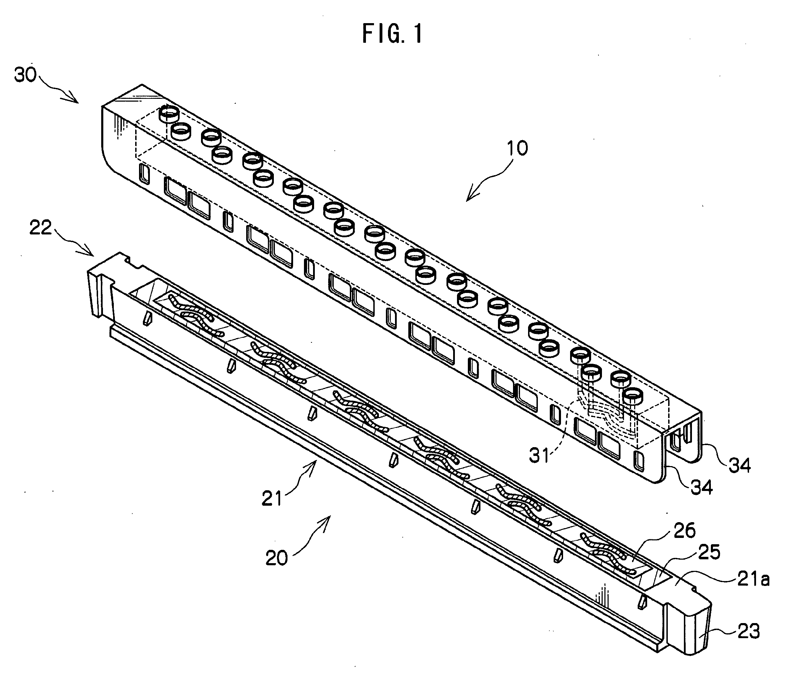

Fig. 1 is an exploded perspective view of a sensor chip 10 for use in measurement utilizing SPR. In Fig. 1, numeral 10 designates a sensor chip, 20 a total reflecting prism, 21 a prism body, 25 a metal film, 26 a polymer layer, 30 a flow channel member, and 31 a flow channel. The sensor chip 10 is useful for a biosensor equipped with an optical system not shown and a control section including a calculating section and constitutes.
The sensor chip 10 is constituted by the total reflecting prism 20 which is a transparent dielectric body and the flow channel member 30 attached onto the total reflecting prism 20. On the flow channel member 30 are provided plural flow channels 31 in the longitudinal direction. Additionally, the bottom shape of each flow channel 31 is not particularly limited as long as each of the flow channels 31 has a cross-section as definite as possible. For example, the bottom shape may be a straight linear shape, a meandering shape, or an arc shape.

The total reflecting prism 20 is constituted by the prism body 21 formed in a long, trapezoidal pillar form, a gripper part 22 provided on one end of the prism body 21, and a projecting part 23 provided on the other end of the prism body 21. This total reflecting prism 20 is formed by, for example, molding according to the extrusion method, wherein the prism body 21, the gripper part 22, and the projecting portion 23 are molded in an integrated manner.

The prism body 21 has an almost trapezoidal cross-section wherein the upper side is longer than the lower side, and collects light irradiated from the side of the bottom surface on a upper surface 21a. On the upper surface 21 a of the prism body 21, the metal film 25 for exciting SPR is provided. The metal film 25 has a rectangular shape in such a way that it faces each flow channel 31 of the flow channel member 30, and is formed by, for example, vapor deposition. For the metal film 25, there may be used, for example, gold or silver, and the film thickness is, for example, 50 nm. The film thickness of the metal film 25 is suitably selected depending upon the material of the metal film 25 and the wavelength of the light which is irradiated upon measurement.

On the metal film 25, the polymer layer 26 is provided. The polymer layer 26 has a binding group for immobilizing a physiologically active substance. The physiologically active substance is immobilized to the metal film 25 via the polymer layer 26.
The polymer layer 26 is a layer for immobilizing the physiologically active substance and can be constituted by self-assembled monolayer-forming molecules, a hydrophilic polymer, a hydrophobic polymer or a combination thereof. It is particularly preferred to use the hydrophilic polymer for the polymer layer 26. According to a particularly preferred embodiment, it can be constituted by a combination of self-assembled monolayer-forming molecules and a hydrophilic polymer.
In addition, it is also possible to coat the metal film 25 with an organic layer having an amino group, and then allow the organic layer to react with a polymer having an activated binding group. Thus, a hydrogel of the polymer layer 26 capable of immobilizing the physiologically active substance can conveniently be produced. As a method for coating the metal film 25 with the organic layer having an amino group, a method known in the art may be employed but, in view of convenience of the procedures, a coating method using self-assembled monolayers (SAMs) is preferred.

A method of coating metal films with self-assembled monolayers (SAMs) has been intensively studied by Professor Whitesides at Harvard University, and the details thereof are described in Chemical Review, 105, 1103-1169 (2005), for example. When gold is used as metal, an alkanethiol derivative represented by formula A-1 (wherein n represents an integer from 3 to 20, and X represents a functional group) is used as an organic layer-forming compound, so that a monomolecular film having orientation can be formed in a self-assemble manner, based on an Au-S bond and the van der Waals force between alkyl chains. A self-assembled monolayer is produced by an extremely easy method, which comprises immersion of a gold substrate in a solution of an alkanethiol derivative. Specially, for example, by forming a self-assembled monolayer using a compound wherein X =NH₂. in formula A-1, it becomes possible to coat a gold surface with an organic layer having an amino group.

HS(CH₂)ₙX A-1

Alkanethiol having an amino group at the terminus thereof wherein X=NH₂ in formula A-1 may be either a compound wherein a thiol group is connected with an amino group via an alkyl chain (formula A-2) (in formula A-2, n represents an integer from 3 to 20), or a compound obtained by allowing alkanethiol having a carboxyl group at the terminus thereof (formulas A-3 and A-4) to react with an excessive amount of hydrazide or diamine. Such a reaction between alkanethiol having a carboxyl group at the terminus thereof and an excessive amount of hydrazide or diamine may be carried out in a solution state. Otherwise, after alkanethiol having a carboxyl group at the terminus thereof has been allowed to bind to the surface of a substrate, an excessive amount of hydrazide or diamine may be allowed to react therewith.

HS(CH₂)ₙNH₂ A-2

HS(CH₂)ₙCOOH A-3

HS(CH₂)ₙ(OCH₂CH₂)ₘOCH₂COOH A-4

Additionally, in the above general formula A-2, the number of repeating alkyl groups, n, represents an integer of from 3 to 20, more preferably an integer of from 3 to 16, particularly preferably an integer of from 4 to 8. Also, in the above general formula A-3, the number of repeating alkyl groups, n, represents an integer of from 3 to 20, more preferably from 3 to 16, particularly preferably from 4 to 8. Further, in the above general formula A-4, the number of repeating alkyl groups, n, each independently represents an integer of from 1 to 20, preferably from 3 to 20, more preferably from 3 to 16, most preferably from 4 to 8.
When the alkyl chain of the alkanethiol derivative is long, formation of the self-assembled monolayer becomes easy and, when the alkyl chain is short, water solubility does not decrease and the handling is not spoiled.
Also, in the above general formula A-4, the number of repeating ethylene oxide groups, m, each independently represents an integer of from 1 to 20, preferably from 3 to 20, more preferably from 3 to 16, most preferably from 4 to 8.

As a large excess diamine to be reacted with the alkanethiol (general formula A-3 or A-4) having a carboxyl group at the end of the molecule, any diamine compound may be used but, in the case of using for the surface of the immobilization substrate for a biosensor, a water-soluble diamine is preferred. Specific examples of a water-soluble diamine that can be preferably used in the present invention may include: aliphatic diamines such as ethylenediamine, tetraethylenediamine, octamethylenediamine, decamethylenediamine, piperazine, triethylenediamine, diethylenetriamine, triethylenetetramine, dihexamethylenetriamine, or 1,4-diaminocyclohexane, and the like; aromatic diamines such as paraphenylene diamine, metaphenylenediamine, paraxylylenediamine, metaxylylenediamine, 4,4'-diaminobiphenyl, 4,4'-diaminodiphenylmethane, 4,4'-diaminodiphenylketone, or 4,4'-diaminodiphenylsulfonic acid, and the like. From the viewpoint of the improvement of hydrophilicity on the surface of the immobilization substrate for a biosensor, a compound wherein two amino groups are connected with each other via an ethylene glycol unit (formula A-5) may also be used. A diamine which can be used is preferably ethylenediamine or compound represented by formula A-5 wherein n and m independently represent an integer of from 1 to 20, and more preferably ethylenediamine or 1,2-bis(aminoethoxy) ethan (a compound having n=2 and m=1 in formula A-5).

H₂N(CH₂)ₙ(OCH₂CH₂)ₘO(CH₂)ₙNH₂ A-5

Alkanethiol having an amino group can also form a self-assembled monolayer by itself. Otherwise, such alkanethiol can be mixed with another alkanethiol, so as to form a self-assembled monolayer. When such an alkanethiol is used as a surface of the immobilization substrate for a biosensor, a compound capable of suppressing non-specific adsorption of a physiologically active substance is preferably used as another alkanethiol. Such a self-assembled monolayer capable of suppressing non-specific adsorption of a physiologically active substance has been studied in detail by the aforementioned Professor Whitesides et al. Professor Whitesides et al. have reported that a self-assembled monolayer formed from alkanethiol having a hydrophilic group is effective for suppression of non-specific adsorption (Langmuir, 17, 2841-2850, 5605-5620, 6336-6343 (2001)). As alkanethiol forming a mixed monomolecular film together with alkanethiol having an amino group for forming a polymer layer 26, a compound described in the aforementioned article can be preferably used. In terms of excellent ability to suppress non-specific adsorption and availability, examples of such alkanethiol forming a mixed monomolecular film together with alkanethiol having an amino group that is preferably used may include alkanethiol having a hydroxyl group (formula A-6) and alkanethiol having an ethylene glycol unit (formula A-7) (in formula A-6, n represents an integer of from 3 to 20, and in formula A-7, n and m independently represent an integer of from 1 to 20). Preferably, n in the formula A-6 is an integer of 5 and more, more preferably an integer of 10 and more, further more preferably an integer of from 10 to 30, most preferably an integer of from 6 to 16.

HS(CH₂)ₙOH A-6

HS(CH₂)ₙ(OCH₂CH₂)ₘOH A-7

In the case where a self-assembled monolayer is formed by mixing the alkanethiol having an amino group with other alkanethiol, the number of repeating alkyl groups, n, in the general formulae A-2 to A-4 is an integer of from 4 to 20, preferably from 4 to 16, most preferably from 4 to 16.. Likewise, in the case where a self-assembled monolayer is formed by mixing the alkanethiol having an amino group with other alkanethiol, the number of repeating alkyl groups, n, in the general formulae A-6 and A-7 is an integer of from 3 to 16, preferably from 3 to 12, most preferably from 3 to 8.

In the present invention, it is possible to mix alkanethiol having an amino group with alkanethiol having a hydrophilic group at any given ratio. When the ratio of alkanethiol having an amino group is small, the binding amount of a polymer that contains an active esterified carboxyl group is decreased. When the ratio of alkanethiol having a hydrophilic group is small, ability to suppress non-specific adsorption is reduced. Accordingly, the mixing ratio between alkanethiol having an amino group and alkanethiol having a hydrophilic group is preferably from 1/1 to 1/1,000,000, more preferably from 1 to 1/1,000, and further more preferably from 1 to 1/10. From the viewpoint of a reduction in steric hindrance occurring during a reaction with a polymer containing an active esterified carboxyl group, the molecular length of alkanethiol having an amino group is preferably longer than that of alkanethiol having a hydrophilic group.

As alkanethiol described above, a compound synthesized based on the summary of Professor Grzybowski at Northwestern University (Curr. Org. Chem., 8, 1763-1797 (2004)) and the cited documents thereof may be used, or a commercially available compound may also be used. Such compounds are available from Dojindo Laboratories, Aldrich, SensoPath Technologies, Frontier Scientific Inc., etc. A disulfide compound that is the oxidation product of alkanethiol can be used as with alkanethiol in the present invention.

As hydrophilic polymers which can be used in the invention, there can be illustrated gelatin, agarose, chitosan, dextran, carrageenan, alginic acid, starch, cellulose or the derivatives thereof such as carboxymethyl derivative, water-swellable organic polymers such as polyvinyl alcohol, polyacrylic acid, polyacrylamide, polyethylene glycol and the derivatives thereof.

As hydrophilic polymers which can be used in the invention, there are further illustrated carboxyl group-containing synthetic polymers and carboxyl group-containing polysaccharides. Examples of such a carboxyl group-containing synthetic polymer may include polyacrylic acid, polymethacrylic acid, and their copolymers such as a methacrylic acid copolymer, an acrylic acid polymer, an itaconic acid copolymer, a crotonic acid copolymer, a maleic acid copolymer, a partially esterified maleic acid copolymer, and a polymer having a hydroxyl group to which an acid anhydride is added, which are described in JP-ANo.59-53836, p.3, right and upper column, line 2 to p.6, left and lower column, line 9 and JP-A No.59-71048, p.3, left and lower column, line 1 to p.5, left and upper column, line 3. Such a carboxyl group-containing polysaccharide may be any one selected from an extract from natural plants, a product obtained by fermentation by microorganisms, a synthetic product obtained by enzymes, and a chemically synthetic product. Specific examples of such a carboxyl group-containing polysaccharide may include hyaluronic acid, chondroitin sulfuric acid, heparin, dermatan sulfate, carboxymethyl cellulose, carboxyethyl cellulose, cellouronic acid, carboxymethyl chitin, carboxymethyl dextran, and carboxymethyl starch, and the like. As a carboxyl group-containing polysaccharide, a commercially available product can be used. Specific examples of such a carboxyl group-containing polysaccharide may include CMD, CMD-L and CMD-D40 (manufactured by Meito Sangyo Co., Ltd.), which are carboxymethyl dextrans, carboxymethylcellulose sodium (manufactured by Wako Pure Chemical Industries, Ltd.), and sodium alginate (manufactured by Wako Pure Chemical Industries, Ltd.), and the like.

A polymer containing a carboxyl group is preferably a polysaccharide containing a carboxyl group, and more preferably carboxymethyl dextran.

The molecular weight of the hydrophilic polymer containing a carboxyl group used in the present invention is not particularly limited. The weight average molecular weight is preferably from 1,000 to 5,000,000, more preferably from 10,000 to 2,000,000, and further more preferably from 100,000 to 1,000,000. When the weight average molecular weight is smaller than the above described range, the amount of a physiologically active substance immobilized becomes small. When the weight average molecular weight is larger than the above described range, it causes a high solution viscosity, and it thereby becomes hard to deal with it.

The hydrophilic polymer as described above may be immobilized to the substrate via the self-assembled monolayer or the hydrophobic polymer as described in this specification, or may directly be formed on the substrate from a monomer-containing solution. In addition, the hydrophilic polymer may be cross-linked. Cross-linking of the hydrophilic polymer is obvious to those skilled in the art.

The hydrophilic polymer immobilized to the sensor surface has a thickness in an aqueous solution of preferably from 1 nm to 300 nm. In case when the thickness is too small, there results a decreased amount of immobilized physiologically active substance and a decreased thickness of the hydrated layer on the sensor surface, which makes difficult the detection of interaction between the physiologically active substance and a test substance due to denaturation of the physiologically active substance itself. In case when the thickness is too large, a test substance is inhibited from distributing into the polymer film and, particularly when the interaction is detected from the opposite side to the hydrophilic polymer-immobilized surface of the sensor substrate, the distance from the detection surface to the interaction-forming part becomes so long that detection sensitivity is decreased. The thickness of the hydrophilic polymer in an aqueous solution can be evaluated by, for example, AFM or ellipsometry.

Also, in the present invention, the immobilization amount of the hydrophilic polymer immobilized to the sensor surface is preferably from 3 ng/mm² to 30 ng/mm² (from 3x10⁻⁶ pmol/mm³ to 30x10⁻⁶ pmol/mm³), more preferably from 3 ng/mm² to 20 ng/mm² (from 3x10⁻⁶ pmol/mm³ to 20x10⁻⁶ pmol/mm³), most preferably from 3 ng/mm² to 15 ng/mm² (from 3x10⁻⁶ pmol/mm³ to 15x10⁻⁶ pmol/mm³). Alternatively, as the thickness of the hydrophilic polymer, the thickness is preferably from 3 nm to 30 nm, more preferably from 3 nm to 20 nm, most preferably from 3 nm to 15 nm.

As the immobilization amount of the hydrophilic polymer, values obtained by various film thickness-measuring methods or weight-measuring methods may be used. Examples of the film thickness-measuring methods include scanning probe microscopy such as atomic force microscopy (AFM) and scanning tunneling microscopy, electron microscopy such as transmission electron microscopy (TEM), scanning electron microscopy (SEM), and scanning transmission electron microscopy (STEM), and ellipsometry.

The polymer included in polymer layer 26 of the present invention preferably reacts with a thin film substrate. As a method for forming a thin film on a substrate, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a dye coating method, a dip coating method, a knife coating method, a blade coating method, a flow coating method, a roll coating method, a wire-bar coating method, and a transfer printing method. These methods for forming a thin film are described in "Progress in Coating Technology (Coating Gijutsu no Shinpo)" written by Yuji Harazaki, Sogo Gyutsu Center (1988); "Coating Technology (Coating Gijutsu)" Technical Information Institute Co., Ltd. (1999); "Aqueous Coating Technology (Suisei Coating no Gijutsu)" CMC (2001); "Evolving Organic Thin Film: Edition for Deposition (Shinka-suru Organic Thin Film: Seimaku hen)" Sumibe Techno Research Co., Ltd. (2004); "Polymer Surface Processing Technology (Polymer Hyomen Kako Gaku)" written by Akira Iwarnori, Gihodo Shuppan Co., Ltd. (2005); and the like. As the method for forming a thin film on a substrate of the present invention, a spray coating method or a spin coating method is preferable. Further, a spin coating method is more preferable. This is because it allows a coating film having a controlled film thickness to be readily produced.
In the case of coating the hydrophilic polymer by employing these methods, the polymer concentration is preferably from 0.005% by weight to 5% by weight, more preferably from 0.05% by weight to 3% by weight, most preferably from 0.1 to 2% by weight.

Also, the hydrophobic polymer to be used as a polymer for constituting the polymer layer 26 is a high-molecular compound having no water-absorbing properties and has a solubility for water (25°C) of 10% by weight or less, more preferably 1% by weight or less, most preferably 0.1 % by weight or less.

As hydrophobic monomers for forming the hydrophobic polymer, proper ones may arbitrarily be selected from among, for example, vinyl esters, acrylic acid esters, methacrylic acid esters, olefins, styrenes, crotonic acid esters, itaconic acid diesters, maleic acid diesters, fumaric acid diesters, allyl compounds, vinyl ethers, and vinyl ketones. The hydrophobic polymer may be a homopolymer composed of one kind of monomer or a copolymer composed of two or more kinds of monomers.
Examples of the hydrophobic polymer which can preferably be used in the invention include polystyrene, polyethylene, polypropylene, polyethylene terephthalate, polyvinyl chloride, polymethyl methacrylate, polyester, and nylon, and the like.

Coating of the hydrophobic polymer on the substrate can be conducted in a conventional manner. For example, coating can be conducted by a spin coating method, an air-knife coating method, a bar coating method, a blade coating method, a slide coating method, a curtain coating method, a spray coating method, a vacuum deposition method, a casting method, or a dipping method.
The coating thickness of the hydrophobic polymer is not particularly limited, but is preferably from 0.1 nm to 500 nm, particularly preferably from 1 nm to 300 nm. The weight-average molecular weight of the hydrophobic polymer is not particularly limited, but is preferably in the range of from 10,000 to 50,000,000.

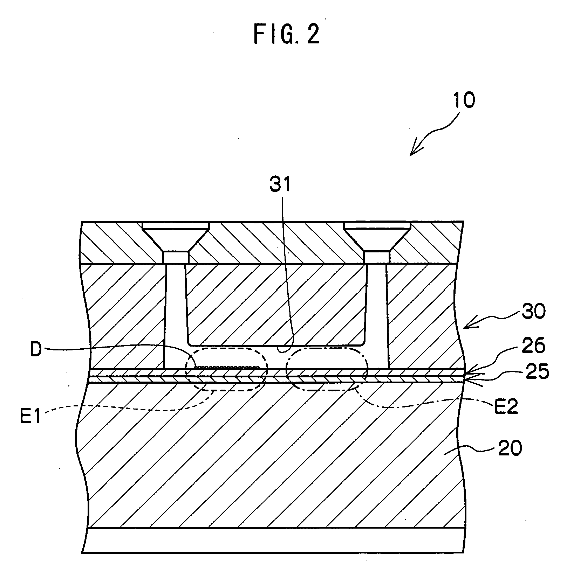

The flow channel member 30 can be constituted by a soft, elastically deformable material, for example, amorphous polyolefin elastomer. Constituting the flow channel member 30 by such elastically deformable material serves to enhance tightness to the total reflecting prism 20 and ensure tight sealing of the flow channel 31 constituted between the member and the total reflecting prism 20.
As is shown in Fig. 2, a measurement region E1 where the physiologically active substance (D in Fig. 2) is immobilized and a reference region E2 where the physiologically active substance is not immobilized are provided in the same flow channel 31.

In the present invention, before attaching the flow channel member 30 to the metal film 25 and the polymer layer 26 described above, the activation treatment using an organic solvent and an activating agent is performed on a part of the polymer layer 26. Thus, there are formed a first surface (in some cases also referred to as "surface 1"), which is activated so as to immobilize the physiologically active substance, and a second surface (in some cases also referred to as "surface 2") which has not been subjected to the activation treatment. Thereafter, the flow channel member 30 is attached to these surfaces to obtain the flow channel 31 having the first surface and the second surface.
Additionally, in the sensor unit 10, the first surface and the second surface are provided, one for each, in each flow channel 31. However, the number of each of the first surface and the second surface to be provided in one communicating flow channel 31 is not limited to one. Also, as to the number of the flow channel member 30, plural of them may be provided.

In the invention, as the activating agent, carbodiimide derivatives, nitrogen-containing compounds, phenol derivatives, and morpholine derivatives are preferably used independently or in combination of two or more thereof in view of activating efficiency.

The carbodiimide derivatives are preferably water-soluble carbodiimides, more preferably compounds (I-1) to (I-3) described below, particularly preferably 1-ethyl-3-(3-dimethylaminopropyl)carbodiimide hydrochloride.
As to the mixing ratio of the carbodiimide derivative as the activating agent, a common mixing ratio employed in this use can be employed as such but, in view of sufficiently immobilizing the polymer, the mixing molar ratio of the carbodiimide derivative with respect to the functional group, for example, carboxyl group in the polymer layer 26 is preferably from 1x10⁻⁴ to 1.

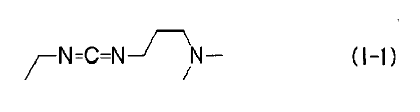

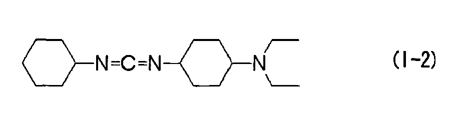

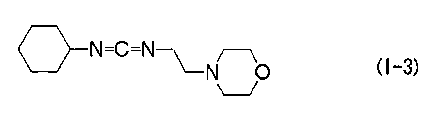

As the nitrogen-containing compounds, there can specifically be illustrated those nitrogen-containing compounds which are represented by the following general formula (IIa) or (IIb) [wherein each of R¹ and R² independently represents a carbonyl group, a carbon atom or a nitrogen atom, which may have a substituent, R¹ and R² may be connected to each other to form a 5- or 6-membered ring, A represents a carbon atom or a phosphorus atom, which has a substituent, M represents an (n-1)-valent element, and X represents a halogen atom].

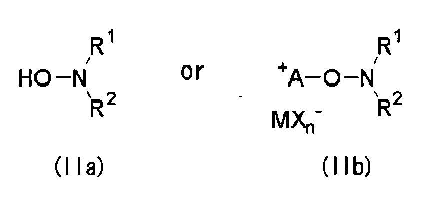

In the above formulae, each of R¹ and R² independently represents a carbonyl group, a carbon atom or a nitrogen atom, which may have a substituent and, preferably, R¹ and R² are connected to each other to form a 5- or 6-membered ring. Particularly preferably, there are provided hydroxysuccinic acid, hydroxyphthalic acid, 1-hydroxybenzotriazole, 3,4-dihydroxy-3-hydroxy-4-oxo-1,2,3-benzotriazine, and the derivatives thereof.

Also, nitrogen-containing compounds shown by the following compounds IIc to IIe can preferably be used.

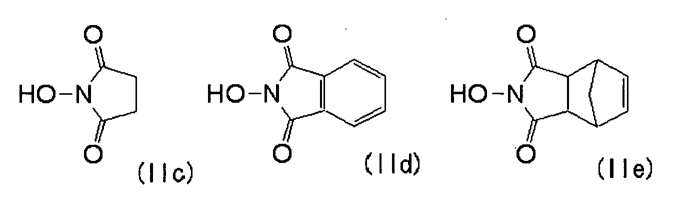

Also, as the nitrogen-containing compounds, compounds represented by the following general formula (III) [wherein each of Y and Z independently represents CH or nitrogen atom] may preferably be used.

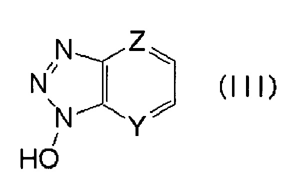

Also, as the nitrogen-containing compounds represented by the above general formula (III), the following compounds (III-1) to (III-3) may specifically be used.

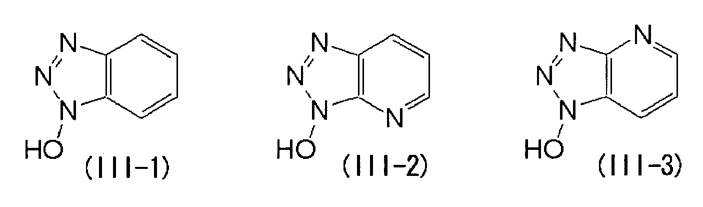

Also, as the nitrogen-containing compound, the following compound (III-4) may preferably be used as well.

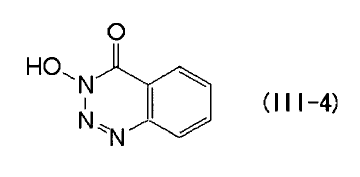

Also, as the nitrogen-containing compounds, compounds represented by the following general formula (IV) [wherein A represents a carbon atom or a phosphorus atom, which has a substituent, each of Y and Z independently represents CH or a nitrogen atom, M represents an (n-1)-valent element, and X represents a halogen atom] may preferably be used.

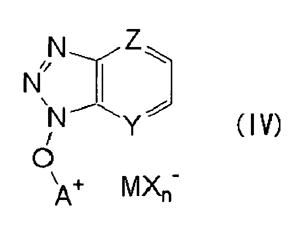

Here, as the substituent for the carbon atom or phosphorus atom represented by A, an amino group having a substituent is preferred. For example, a dialkylamino group such as a dimethylamino group or a pyrrolidino group is preferred. Examples of the (n-1)-valent element represented by M include a phosphorus atom, a boron atom, and an arsenic atom, with a phosphorus atom being preferred. Examples of the halogen atom represented by X include a fluorine atom, a chlorine atom, a bromine atom, and an iodine atom, with a fluorine atom being preferred.

Specific examples of the nitrogen-containing compounds represented by the general formula (IV) include the following compounds (IV-1) to (IV)-6.

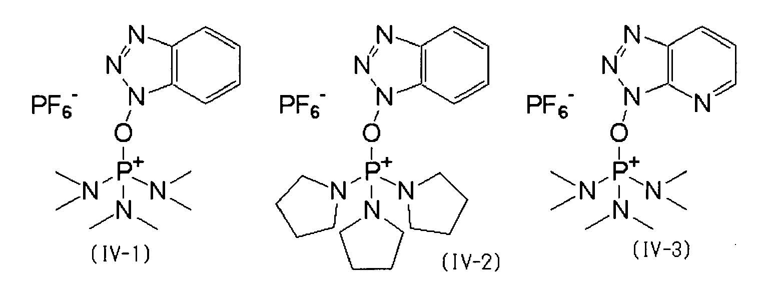

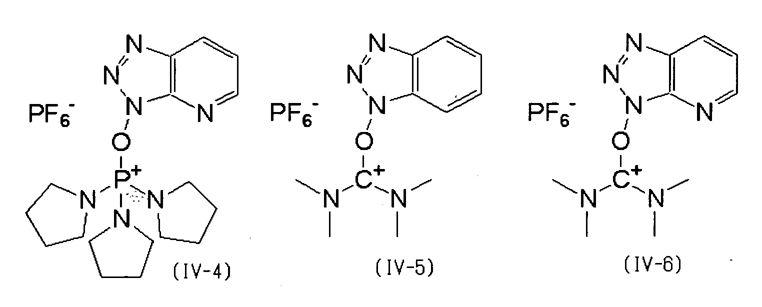

Also, as the nitrogen-containing compounds, compounds represented by the following general formula (V) [wherein A represents a carbon atom or a phosphorus atom, which has a substituent, M represents an (n-1)-valent element, and X represents a halogen atom] may preferably be used.

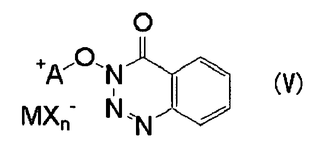

Specifically, the following compound (V-1) may be used.

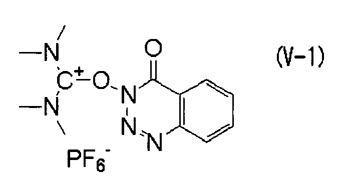

As to the mixing ratio of the nitrogen-containing compound as the activating agent, a common mixing ratio employed in this use can be employed as such but, in view of sufficiently immobilizing the polymer, the mixing molar ratio of the nitrogen-containing compound with respect to the functional group, for example, carboxyl group in the polymer layer 26 is preferably from 1x10⁻⁷ to 1.

In view of sufficiently immobilizing the polymer, the phenol derivative is preferably a phenol derivative having an electron-withdrawing group, with the electron-withdrawing group preferably having a σ value of 0.3 or more in view of sufficiently immobilizing the polymer. Specifically, the following compounds (VI-1) to (VI-4) may be used. Additionally, in (VI-4), X⁻ represents an anion.

As to the mixing ratio of the phenol derivative as the activating agent, a common mixing ratio employed in this use can be employed as such but, in view of sufficiently immobilizing the polymer, the mixing molar ratio of the phenol derivative with respect to the functional group, for example, carboxyl group in the polymer layer 26 is preferably from 1x10⁻⁴ to 1.

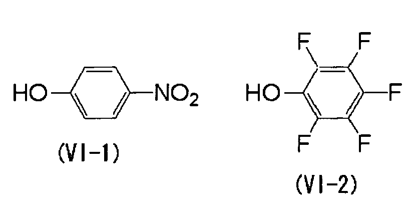

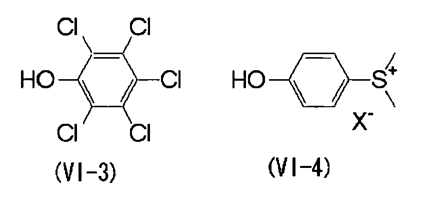

As the morpholine derivative, there can be preferably illustrated the following compound (VII). As to the mixing ratio of the morpholine derivative as the activating agent, a common mixing ratio employed in this use can be employed as such but, in view of sufficiently immobilizing the polymer, the mixing molar ratio of the morpholine derivative with respect to the functional group, for example, carboxyl group in the polymer layer 26 is preferably from 1x10⁻⁴ to 1.
The morpholine compound may be used independently or may be used in combination with the carbodiimide derivative, the nitrogen-containing compound or the phenol derivative.

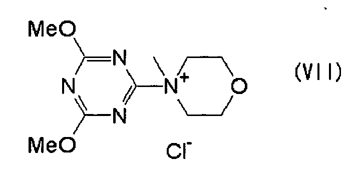

The above-described carbodimide derivatives, the nitrogen-containing compounds, the phenol derivatives, and the morpholine derivatives may be used independently but, in view of sufficiently immobilizing the polymer, it is preferred to use two or more kinds of them in combination. Combined use of the carbodiimide derivative and the nitrogen-containing compound is more preferred.

Further, as an approach for activating carboxylic acid in the polymer containing a carboxyl group, there can also preferably be employed a method described in JP-A No.2006-58071, paragraph Nos. [0011] to [0022] (i.e., a method of forming a carboxylic acid amido group by activating the carboxyl group existing on the surface of the substrate with any compound selected from a uronium salt, a phosphonium salt or a triazine derivative having a particular structure) and a method described in JP-A No.2006-90781, paragraph Nos. [0011] to [0019] (i.e., a method of forming a carboxylic acid amido group by activating the carboxylyl group existing on the surface of the substrate with a carbodiimide derivative or the salt thereof, converting the activated group to an ester group using a nitrogen-containing hetero aromatic compound having a hydroxyl group, a phenol derivative having an electron-withdrawing group or an aromatic compound having a thiol group, and then reacting with an amine).

It is to be noted that the aforementioned uronium salt, phosphonium salt, and triazine derivative, which have a specific structure, described in Japanese Patent Application No. 2004-238396(JP Patent Publication (Kokai) No.2006-58071A), mean the uronium salt represented by the following formula 1, the phosphonium salt represented by the following formula 2, and the triazine derivative represented by the following formula 3, respectively.

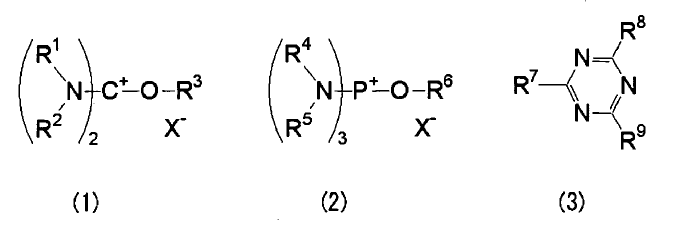

In the general formulae (1) to (3), each of R¹ and R² independently represents an alkyl group containing from 1 to 6 carbon atoms or, when taken together, represents an alkylene group containing from 2 to 6 carbon atoms to form a ring together with a nitrogen atom, R³ represents an aromatic group containing from 6 to 20 carbon atoms or a hetero ring group containing at least one or more hetero atoms, and X⁻ represents an anion. In the general formula (2), each of R⁴ and R⁵ independently represents an alkyl group containing from 1 to 6 carbon atoms or, when taken together, represents an alkylene group containing from 2 to 6 carbon atoms to form a ring together with a nitrogen atom, R⁶ represents an aromatic group containing from 6 to 20 carbon atoms or a hetero ring group containing at least one or more hetero atoms, and X⁻ represents an anion. In the general formula (3), R⁷ represents an onium group, and each of R⁸ and R⁹ independently represents an electron-withdrawing group.

The organic solvent to be used for the activation treatment together with the activating agent may be any organic solvent that does not seriously spoil the function of the above-described activating agent which activates the functional group of the polymer and, in view of reactivity between the activating agent and carboxyl group, those organic solvents are preferred which are water-miscible to a suitable degree. Here, the term "water-miscible organic solvent" means a solvent which has a solubility for water at 25°C of 5% by volume or more. Examples of such organic solvent include methanol, ethanol, propanol, isopropanol, butanol, isobutanol, 2-butanol, methyl acetate, ethyl acetate, N,N-dimethylformamide, dimethylsulfoxide, acetone, methyl ethyl ketone, ethylene glycol, glycerin, methyl cellosolve, cellosolve, propyl cellosolve, butyl cellosolve, tetrahydrofuran, dioxane, and acetonitrile. Of these, methanol, ethanol, ethyl acetate, N,N-dimethylformamide, dimethylsulfoxide, acetone, methyl ethyl ketone, and acetonitrile are more preferred due to high miscibility with water. When using for the activation treatment, these organic solvents may be used as a mixture with water or other solvent. In the case of mixing with water or other solvent, the content of the organic solvent may be at least 10% by volume or more, and is preferably 30% by volume or more, most preferably 50% by volume or more.

The activation treatment by using the organic solvent and the activating agent may be carried out by using the activating agent and the organic solvent at the same time or successively as long as the functional group in the polymer layer 26 is imparted with the ability of immobilizing the physiologically active substance but, in view of the activating efficiency, it is preferred to constitute the activation treatment by a first treatment using the activating agent and a subsequent second treatment using the organic solvent. The activating agent is surmised to activate the functional group in the polymer layer 26 so that the physiologically active substance can be immobilized, whereas the organic solvent is surmised to maintain the immobilizing ability of the activated functional group of the polymer layer 26.

The treatment with the organic solvent may be carried out by supplying it, together with the activating agent or after the treatment with the activating agent, to the first surface of the polymer layer 26 through dropwise application. For ensuring maintainance of the immobilizing ability of the activated functional group, it is preferred to repeat the dropwise application and removal several times. The supplying amount to the first surface can properly be selected depending upon the area size of the first surface.
The supplying amount of the organic solvent varies depending upon kind of the solvent to be used and kind of the selected activating agent but, generally, can be from 0.1 mm to 5 mm in thickness with respect to the area size of the first surface. In view of reactivity and evaporation, the supplying amount can preferably be from 0.3 mm to 3 mm.

The activation treatment is carried out with respect to a part of the polymer layer 26. Thus, on polymer layer 26, at the same time as forming the first surface which has been subjected to the activation treatment, the second surface is formed as a surface which has not been subjected to the activation treatment.

In order to form the first surface on one part of the polymer layer 26 and the second surface on other part thereof, it suffices to subject a part of the surface of the polymer layer 26 to the activation treatment with both the activating agent and the organic solvent. It is also possible to provide, between the first surface and the second surface, a third surface more hydrophobic than the first and second surfaces. Thus, the activation treatment using the activating agent and the organic solvent can be carried out effectively due to the hydrophobic effect based on the difference in surface characteristics between the first and second surfaces and the third surface. In this occasion, the hydrophobic third surface may be formed by, for example, forming a hydrophobic polymer on the polymer layer 26. As such hydrophobic polymer, there can be illustrated those which have been described hereinbefore.

As methods for forming the hydrophobic polymer, there are illustrated a method of directly immobilizing the hydrophobic polymer on the polymer layer 26 through chemical reaction; and a method of dissolving the hydrophobic polymer in an organic solvent and coating the solution on the polymer layer 26.
Additionally, it suffices that such third surface is provided between the first surface and the second surface, and the third surface may be provided around the first surface or around the second surface.

Also, the surface of the substrate may be made non-continuous. For example, it is possible to form the polymer layer 26 on a non-continuous metal film 25 which has been previously made non-continuous, and subsequently perform the activation treatment. A non-continuous metal film 25 such as this, or the polymer layer 26, may alternatively be formed by uniformly forming one of these, and then forming a groove or the like therein, or attaching a mask or the like thereto, to obtain a difference in level. Alternatively, a groove or a mask may previously be provided on the total reflecting prism 20, followed by forming the metal layer 25 or the polymer layer 26 thereon. Thus, the activation treatment may be easily performed on only the first surface.
Further, when the first surface and the second surface are non-continuous, the first surface becomes more independent from the second surface; therefore, each surface may be subjected to one or more individual treatments as necessary. Such treatments may be the same or different, and the treatment or reaction performed may be appropriately selected depending upon the desired purpose.

In the present invention, it is preferable to dry the substrate after the activation treatment. Thus, the functional group of the polymer layer 26 activated by the activation treatment may be protected by the dried organic solvent to effectively maintain the immobilizing ability of the functional group.
Drying includes natural drying wherein the polymer layer 26 is allowed to stand after dropwise applying the activating agent or the organic solvent, and intentional drying wherein the drying rate of the substrate is increased by heating or by blowing air. In particular, drying after dropwise application of the organic solvent after the first step of using the activating agent serves to effectively prevent deterioration of the activating agent, and is thus preferable.

When the flow channel member 30 is attached so as to face the thus obtained first and second surfaces, a flow channel 31 having both the first surface and the second surface is obtained. Upon supplying a solution containing a physiologically active substance (physiologically active substance-containing solution) to flow channel 31, the physiologically active substance comes into contact with the polymer layer 26 which has been subjected to the activation treatment, thus immobilizing the physiologically active substance and obtaining the measurement region E1. At this time, the physiologically active substance-containing solution is also simultaneously supplied to the second surface. However, since the activation treatment has not been performed on the second surface, the physiologically active substance is not immobilized on the second surface, thereby obtaining reference area E2 at which the physiologically active substance is not immobilized.

In order to immobilize the physiologically active substance by using the physiologically active substance-containing solution, it suffices to bring the physiologically active substance into contact with the first surface, which may be achieved by supplying the physiologically active substance-containing solution to the flow channel 31 or by delivering the physiologically active substance-containing solution after supplying the solution to thereby continuously perform the immobilizing treatment.

A physiologically active substance immobilized is not particularly limited, as long as it interacts with a measurement target. Examples of such a substance may include an immune protein, an enzyme, a microorganism, a nucleic acid, a low molecular weight organic compound, a non-immune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant Staphylococcus aureus, antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55, 111 and 157 among enteropathogenic Escherichia coli.

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant Staphylococcus aureus, antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as Escherichia coli can be used.
As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.
As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor.
Examples of an immunoglobulin-binding protein used herein may include protein A, protein G, and a rheumatoid factor (RF).
As a sugar-binding protein, for example, lectin is used.
Examples of fatty acid or fatty acid ester may include stearic acid, arachidic acid, behenic acid, ethyl stearate, ethyl arachidate, and ethyl behenate.

The physiologically active substance is supplied to the flowing channel 31 as a physiologically active substance-containing solution prepared by dissolving in an appropriate dissolving liquid. The concentration of the physiologically active substance-containing solution may properly be changed depending upon kind and molecular weight of the target physiologically active substance, but is generally from 0.001 mg/ml to 10 mg/ml:

The immobilization substrate wherein the physiologically active substance is immobilized as described above can be used for detecting and/or measuring a substance which interact with the physiologically active substance.

In the invention, the interaction between the physiologically active substance immobilized to the substrate for a sensor and a test substance is preferably detected and/or measured by a non-electrochemical method. As the non-electrochemical method, there are illustrated the surface plasmon resonance (SPR) measurement technique, a quartz crystal microbalance (QCM) measurement technique, and a measurement technique of using functional surfaces ranging from gold colloid particles to ultra-fine particles.
According to a preferred embodiment of the invention, the immobilization substrate in the invention can be used as an immobilization substrate for surface plasmon resonance which is characterized by being provided with a metal film disposed on a transparent substrate.

An immobilization substrate for surface plasmon resonance is an immobilization substrate used for a surface plasmon resonance biosensor, meaning a member comprising a portion for transmitting and reflecting light emitted from the sensor and a portion for immobilizing a physiologically active substance. It may be fixed to the main body of the sensor or may be detachable.

SPR is a phenomenon defined as follows. When the irradiation light is incident on the metal film 25, two waves are generated. That is, a low energy wave (a so-called evanescent wave) is generated on an emission-side surface of the metal film 57, and a compressional wave (a so-called evanescent wave) is generated on the interface between the total reflecting prism 20 and the metal film 57. When these evanescent waves are identical in wave number, i.e., match in wave number, these waves are in a resonant state. In the resonant state, at least a part of light energy transfers to the surface plasmon resonance, thereby causing a great reduction in the intensity of the light totally reflected by the interface between the metal film 25 and the total reflecting prism 20. That is, SPR is a phenomenon whereby the intensity of the light reflected from the metal film 25 undergoes attenuated total reflection. The evanescent waves are generated on an opposite surface of the metal film 25 to the surface on which the irradiation light is incident when the irradiation light L is incident on the metal film 25 at a specific incident angle equal to or greater than the angle of total reflection.

A dielectric constant, which is expressed by a square of a refraction index, has an effect on the evanescent waves. Due to this, the interaction between the physiologically active substance and the test substance produced on the surface of the metal film 25 causes a difference in dielectric constant, the difference influences the surface plasmon resonance, and the influence can be understood as a change in resonance angle, that is, a change in refraction index.

When the interaction occurs between the physiologically active substance and the test substance contained in the supplied sample solution on the surface of the metal film 25, the dielectric constant of the surface of the metal film 25 changes and the refraction index (angle of resonance) changes. Thus, by selectively supplying different samples (sample solutions) to the physiologically active substance on the surface of the metal film 25, it is possible to measure a change in refraction index at time series and to analyze the intermolecular interaction.

Here, in obtaining refractive index information from dielectricities of the measurement region E1 and the reference area E2, the refractive index information of a test substance in each measurement area E1 can be obtained by correcting the refractive index information of the measurement region E1 by the refractive index information of the reference region E2. As a result, there can be obtained interaction information between the physiologically active substance immobilized to the measurement region E 1 and the test substance.

In the case of using the immobilization substrate of the invention for surface plasmon analysis, the immobilization substrate can be used as part of a surface plasmon measurement apparatus known in the art.

The invention will be specifically described in the following examples. However, the examples are not intended to limit the scope of the invention.

### [Examples]

### [Example 1]

A surface 1 capable of immobilizing a protein and a surface 2 as a reference region are prepared to evaluate the immobilization amount of the protein and the ability of detecting bonding of a compound.

### (1) Preparation of a substrate

A one mM aqueous solution of 6-amino-1-octanethiol, hydrochloride (manufactured by Dojindo Laboratories) was prepared. This solution is designated as Solution A.
Next, a gold thin film was formed by the method described below on the upper surface of a plastic prism by injection-molding ZEONEX (manufactured by Zeon Corporation).
The prism was secured to a substrate holder of a sputtering apparatus, and a vacuum (base pressure of 1x10⁻³ Pa or less) was drawn therein. Thereafter, Ar gas was introduced into the apparatus (1 Pa), and RF (electrical signals of high frequency) power (0.5 kW) was applied to the substrate holder for about 9 minutes with the substrate holder rotated (20 rpm) to plasma-treat the prism surface. Next, introduction of Ar gas was stopped, and a vacuum was drawn therein. Ar gas was again introduced (0.5 Pa), and DC (DC voltage) power (0.2 kW) was applied to a 8-inch Cr target for about 30 seconds with the substrate holder rotated (10 to 40 rpm) to form a 2-nm Cr thin film thereon. Subsequently, introduction of Ar gas was stopped, and a vacuum is drawn again. Ar gas was introduced (0.5 Pa) again to the apparatus, and DC power (1 kW) was applied to a 8-inch Au target for about 50 seconds with the substrate holder rotated (20 rpm) to form an about 50-nm Au thin film thereon.
A 3-mm gap was provided between the Au film corresponding to the surface 1 (corresponding to the measurement region E1) and the Au film corresponding to the surface 2 (corresponding to the reference region E2) by attaching a mask to the substrate holder.
The thus-obtained plastic prism having the Au thin film formed thereon was dipped in Solution A at 40°C for 1 hour, followed by washing 5 times with ultra-pure water.

### (2) Active esterification of CMD (carboxymethyldextran)

After dissolving 20 g of a solution of 1% by weight of CMD (manufactured by Meito Sangyo; weight-average molecular weight: 1,000,000), 20 ml of 10 mM of EDC (1-Ethyl-3-[3-Dimethylaminopropyl]carbodiimide hydrochloride was added thereto, followed by stirring at room temperature for 1 hour.

### (3) Binding reaction of CMD to the substrate

Onto the substrate (plastic prism having the Au thin film formed thereon) prepared in the paragraph (1), 1 ml of the mixed solution of CMD and EDC prepared in the paragraph (2) was applied dropwise, spin-coated at 1,000 rpm for 45 seconds, and allowed to stand at room temperature for 15 minutes to react, thereby immobilizing the carboxymethyl dextran thin film on the substrate having an amino group. Thereafter, the substrate was dipped in a 1 N NaOH aqueous solution for 30 minutes and washed 5 rimes with ultra-pure water to form a CMD film.

### (4) Preparation of a sensor stick

Onto the surface corresponding to the surface 1 on the CMD film prepared in the paragraph (3), 10 pl of each of the activating solutions described in Table 1 was applied dropwise, followed by allowing to stand for 7 minutes. After standing, each of the dropwise added solution was removed by absorption. Then the procedure of dropwise application and absorption of each solvent (10 µl) described in Table 1 to and from the same surface was repeated three times. The thickness upon dropwise addition was about 1 mm. After sufficiently removing the solvent from the surface and drying for 1 hour, a flow channel was attached so that the surface 1 and the surface 2 exist in the same flow channel to obtain sensor sticks 1 to 4. The flow channel had a volume of 10 µl and a bottom area of 1 mm².

### [Example 2]

An Au thin film having no gap between the surface 1 and the surface 2 was formed by changing the mask shape used in the step of the paragraph (1) in Example 1. Sensor stick 5 was obtained by similarly carrying out the steps of (2) to (4) in Example 1 using the solutions described in Table 1.

### [Example 3]

Example 3 relates to immobilization of a protein to the sensor samples obtained in Examples 1 and 2. As the protein, CA (Carbonic anhydrase; manufactured by SIGMA) was used.
After standing for one hour in a state wherein the surface of each sample prepared in Examples 1 and 2 was covered with water, each sample was loaded in a surface plasmon resonance apparatus. After injecting a PBS buffer thereinto to confirm a base line (resonance angle in this occasion being used as a reference point), injection of 0.1 mg/ml of a CA solution (acetic acid buffer; pH: 5.0) and 15-minute standing, next injection of a PBS buffer and 1-minute standing, next injection of a 1 M ethanolamine solution (Biacore) and 7-minute standing, next injection of a PBS buffer and 1-minute standing, next three runs of injection of 10mM of NaOH and 1-minute standing and, finally, injection of a PBS buffer and 1-minute standing were performed. The difference between the resonance angle here and the original resonance angle was used as the amount of immobilized CA. In this context, a resonance angle difference per 1% by volume of DMSO is referred to as an immobilized protein amount of 1,500 RV.

Next, a 10 µM furosemide solution (PBS buffer/DMSO 10% by volume mixed solvent) was prepared, and a PBS buffer/DMSO 10% by volume mixed solvent used as a base and the furosemide solution were injected into the flow channel to measure bonding of furosemide to CA. The value immediately before injection of the furosemide solution was used as a base line. Measurement of furosemide was carried out three times in total to calculate the average value of the binding amount. Additionally, in order to correct the influence of change in the refractive index of the surface 1 and that of the surface 2, (a PBS buffer/DMSO 9.5% by volume mixed solvent) and (a PBS buffer/DMSO 10.5% by volume mixed solvent) were introduced three times to perform calibration. Sensor samples having been left in the laboratory for 14 days were also subjected to the same treatment.

The immobilization amount of CA and the binding amount of furosemide on the surface 1 with each sample are shown in Table 1. Regarding detection of the compound, an amount of 5RV or more is enough to detect. It has been found that, according to the invention, even when time has elapsed, a large amount of protein can be immobilized by a simple method and a low-molecular compound can be measured with good accuracy.

**[Table 1]**

| Sample No. | Gap | Activating Solution | Solvent | Immobilization Amount of CA to Surface 1 (RV) | | Average Binding Amount of Furosemide (RV) | | Note |
|---|---|---|---|---|---|---|---|---|
| | | | | Immediately After | After 14 Days | Immediately After | After 14 Days | |
| 1 | yes | 0.1 M EDC 50 mM NHS | water | 1200 | 180 | 4 | 0 | Comparative Example |
| 2 | yes | 0.1M EDC/ 0.2 mM HOBt | water | 940 | 900 | 2 | 2 | Comparative Example |
| 3 | yes | 0.1M EDC/ 50 mM HOBt* | water | 800 | 900 | 2 | 2 | Comparative Example |
| 4 | yes | 0.1M EDC/ 50 mM HOBt | DMSO | 3600 | 3000 | 17 | 15 | Present Invention |
| 5 | no | 0.1M EDC/ 50 mM HOBt | DMSO | 4300 | 4400 | 8 | 8 | Present Invention |

| | | | | | | | | |
|---|---|---|---|---|---|---|---|---|
| - EDC: 1-(3-Dimethylaminopropyl)-3-ethylcarbodiimide - NHS: N-Hydroxysuccinimide - HOBt: 1-Hydroxybenzotriazole - DMSO: Dimethylsulfoxide *: HOBt is poorly soluble. | | | | | | | | |

All publications, patent applications, and technical standards mentioned in this specification are herein incorporated by reference to the same extent as if such individual publication, patent application, or technical standard was specifically and individually indicated to be incorporated by reference. It will be obvious to those having skill in the art that many changes may be made in the above-described details of the preferred embodiments of the present invention. The scope of the invention, therefore, should be determined by the following claims.

## Claims

1. A method for producing an immobilization substrate for a biosensor, the immobilization substrate having a measurement surface having a physiologically active substance immobilized thereon, and a reference surface not having a physiologically active substance immobilized thereon, the biosensor measuring an interaction, in terms of a change in refractive index, between the physiologically active substance and a test substance in a solution which is supplied to both the measurement surface and the reference surface, the method comprising:
performing an activation treatment on a part of the substrate surface by using an organic solvent and an activating agent, thereby forming, on the substrate, a first surface which is activated such that it can immobilize the physiologically active substance, and forming a second surface which has not been subjected to the activation treatment;
forming a flow channel which includes both the first surface and the second surface by attaching a flow channel member to the substrate; and
supplying the physiologically active substance to the flow channel to bring the first surface into contact with the physiologically active substance, and thereby prepare the measurement surface and the reference surface within the same flow channel.

2. The method according to claim 1, wherein the substrate is a metal surface or a metal film.

3. The method according to claim 2, wherein the metal is at least one selected from the group consisting of gold, silver, copper, platinum, and aluminum.

4. The method according to claim 3, wherein the metal is gold.

5. The method according to claim 2, wherein a polymer layer of self-assembled film-forming molecules, a hydrophilic polymer, a hydrophobic polymer, or a combination thereof is provided on the metal.

6. The method according to claim 2, wherein a hydrophilic polymer is provided on the metal surface or on the metal film.

7. The method according to claim 1, wherein a third surface which is more hydrophobic than both the first surface and the second surface is provided between the first surface and the

8. The method according to claim 2, wherein the substrate surface between the first surface and the second surface is non-continuous.

9. The method according to claim 2, wherein a difference in level is provided between the first surface and the second surface.

10. The method according to claim 1, which includes drying the substrate after the activation treatment.

11. The method according to claim 1, wherein the physiologically active substance is immobilized on the first surface by introducing the physiologically active substance-containing solution into the flow channel.

12. The method according to claim 1, wherein the activation treatment includes a first treatment of using the activating agent and a subsequent second treatment of using the organic solvent.

13. The method according to claim 1, wherein the organic solvent is a water-miscible organic solvent.

14. The method according to claim 1, wherein the organic solvent is at least one selected from the group consisting of methanol, ethanol, propanol, isopropanol, butanol, isobutanol, 2-butanol, methyl acetate, ethyl acetate, N,N-dimethylformamide, dimethylsulfoxide, acetone, methyl ethyl ketone, ethylene glycol, glycerin, methyl cellosolve, cellosolve, propyl cellosolve, butyl cellosolve, tetrahydrofuran, dioxane, and acetonitrile.

15. The method according to claim 1, wherein the activating agent is at least one selected from the group consisting of carbodiimide derivatives, nitrogen-containing compounds, phenol derivatives, and morpholine derivatives.

16. An immobilization substrate produced by the method of claim 1.

17. The immobilization substrate according to claim 16, which is used for non-electrochemical detection.

18. The immobilization substrate according to claim 16, which is used for surface plasmon resonance analysis.
